# EUROPÄISCHE PATENTSCHRIFT

(11) **EP 1 569 551 B1**
(45) Veröffentlichungstag und Bekanntmachung des Hinweises auf die Patenterteilung: **20.02.2008**
(21) Anmeldenummer: 03779542.4
(22) Anmeldetag: 10.12.2003
(51) Int. Cl.: A61B 5/0408

(54) **MEDIZINISCHE ELEKTRODE**
MEDICAL ELECTRODE
ELECTRODE MEDICALE

(30) Priorität: 13.12.2002 AT 18672002
(43) Veröffentlichungstag der Anmeldung: 07.09.2005
(73) Patentinhaber: Leonhard Lang KG, 6020 Innsbruck (AT)
(72) Erfinder: LANG, Burrhus, A-6020 Innsbruck (AT); WILFINGER, Markus, A-6020 Innsbruck (AT)
(74) Vertreter: Torggler, Paul Norbert
(86) Internationale Anmeldenummer: PCT/AT2003/000366
(87) Internationale Veröffentlichungsnummer: WO 2004/054442

(56) Entgegenhaltungen:
- CH-A- 662 717
- DE-C- 19 730 811
- US-A- 4 122 843
- US-A- 4 155 354
- US-A- 4 442 315

## Beschreibung

Die Erfindung betrifft eine medizinische Elektrode für die Ableitung von Signalen, insbesondere zur Signalableitung von der menschlichen Haut, die wenigstens zweischichtig aufgebaut ist, wobei zwischen einer oberen Deckschicht und einer mit dem signalgebenden Subjekt kontaktierbaren Klebeschicht ein vorzugsweise metallischer, mit dem signalgebenden Subjekt verbindbarer Signalleiter angeordnet ist, und die eine Abgrifflasche aufweist, wobei zwischen der Abgrifflasche und dem signalgebenden Subjekt ein vorzugsweise elektrisch isolierendes Abdeckelement angeordnet ist.

Bekannte, auf die Haut aufklebbare Elektroden bestehen aus einer Klebeschicht mit einer Aussparung zur Aufnahme eines elektrisch leitenden Gels zur Kontaktierung mit dem signalgebenden Subjekt, einer oberen Deckschicht und einer dazwischen angeordneten Signalleitschicht und weisen eine Abgrifflasche auf, die von der oberen Deckschicht und der Signalleitschicht gebildet wird. Um zu verhindern, dass die von der Unterseite der Abgrifflasche frei zugängliche Signalleitschicht mit dem signalgebenden Subjekt in Berührung kommt, wodurch das geleitete Signal beeinflusst würde, ist es üblich, auf der dem signalgebenden Subjekt zugewandten Seite der vorzugsweise elektrisch nicht leitenden Klebeschicht ein hautfreundliches Pflasterelement, anzuordnen, das die Abdecklasche überdeckt und somit einen Kontakt zwischen der Signalleitschicht und dem signalgebenden Subjekt im Bereich der Abgrifflasche verhindert.

Aus der DE 197 30 811 C1 ist es weiters bekannt, die Abgrifflasche auf der zum signalgebenden Objekt zugewandten Seite mit einer nichtklebenden Abdeckung zu versehen und auf diese Weise die Signalleitschicht der Elektrode gegenüber äußeren Störeinflüssen abzuschirmen.

Es hat sich jedoch gezeigt, dass die in der Regel aus Kunststoff, beispielsweise aus PVC, PE oder PET, hergestellten Abdeckungen während des Messvorganges eine statische Aufladung erfahren, insbesondere wenn sich das signalgebende Subjekt bewegt, wie dies beispielsweise bei Durchführung eines Belastungs-EKG's der Fall ist. Diese statische Aufladung des Abdeckelementes wirkt sich auf die Signalleitschicht, mit der das Abdeckelement ja direkt in Kontakt steht, aus und beeinflusst auf diese Weise das abgeleitete Signal.

Als weiterer Nachteil an den bekannten medizinischen Elektronen hat es sich herausgestellt, dass die Kunststoffe, die für die Herstellung der Abdeckung verwendet werden, unterschiedlich günstige statische Eigenschaften aufweisen. Von diesem Gesichtspunkt aus betrachtet, eignet sich für die Herstellung der Abdeckung PVC am besten. Hingegen bevorzugt man für die Herstellung der oberen Deckschicht der medizinischen Elektrode, unter anderem aus ökologischen Gründen, PE oder PET. Im Sinne eines einfachen und kostengünstigen Herstellungsverfahrens wäre es sinnvoll, die obere Deckschicht und die Abdeckung einstückig auszubilden, was in der Praxis dazu führt, dass man entweder eine Elektrode mit einer qualitativ hochwertigen oberen Deckschicht, jedoch schlechteren statischen Eigenschaften für die Abdeckung oder eine Elektrode mit einer qualitativ hochwertigen Abdeckung, aber einer minder geeigneten oberen Deckschicht erhält.

Ausgehend von diesem Stand der Technik ist es Aufgabe der Erfindung, eine neuartige medizinische Elektrode anzugeben, die unter Vermeidung der vorbeschriebenen Nachteile eine Abschirmung der Signalleitschicht gegenüber äußeren Störsignalen erlaubt und gleichzeitig kostengünstig herzustellen ist.

Erfindungsgemäß wird diese Aufgabe durch die Merkmale von Anspruch 1 gelöst. Dabei ist vorgesehen, dass wenigstens eine Seite des vorzugsweise flächigen Abdeckelementes elektrisch leitend ausgebildet ist, wodurch verhindert wird, dass sich die Abdeckung statisch aufladet und das abgeleitete Signal beeinflusst bzw. verändert. Dabei kommt es in erster Linie nicht darauf an, wie die elektrisch leitende Seite des Abdeckelementes ausgebildet ist, erfindungswesentlich ist vielmehr der Umstand, dass das Abdeckelement eine leitende Seite aufweist. So kann die elektrisch leitende Seite des Abdeckelementes beispielsweise von einem gitterförmig ausgebildeten, elektrisch leitenden Element oder von einem elektrisch leitfähigen Schaum gebildet sein. Aber auch die Verwendung eines dünnen Metallstreifens oder eines mit metallischen Fäden durchzogenen textilen Flächengebildes wäre ebenso denkbar, wie die Verwendung eines durchgängig leitenden Abdeckelementes - beispielsweise eines Karbonleiters - oder das Aufdrucken einer elektrisch leitenden Schicht auf das Abdeckelement. Durch die elektrisch leitende Ausbildung einer Seite des Abdeckelementes ist es im Gegensatz zum Stand der Technik nicht mehr relevant, welche statischen Eigenschaften der für die Herstellung des Abdeckelementes verwendete Kunststoff aufweist, sodass bei der Herstellung eines einstückig mit der Deckschicht ausgebildeten Abdeckelementes auch statisch weniger geeignete Kunststoffe zum Einsatz gelangen können.

Gemäß einem bevorzugten Ausführungsbeispiel der Erfindung ist das Abdeckelement als vorzugsweise zweilagige Folie mit einer ersten elektrisch isolierenden Trägerschicht und einer zweiten elektrisch leitenden Schicht ausgebildet, wobei die elektrisch isolierende Schicht klebrig ausgebildet sein kann, sodass die Abdeckfolie unabhängig vom übrigen Herstellungsprozess der Elektrode in einfacher Weise auf die dem signalgebenden Subjekt zugewandte Seite der Abdecklasche aufgeklebt werden kann. Gleichzeitig erlaubt es eine derartige Ausbildung des Abdeckelementes, bereits im Umlauf befindliche, herkömmliche medizinische Elektroden mit einem Abdeckelement, das eine elektrisch leitende Seite aufweist, auszustatten.

Damit die elektrisch leitende Seite des Abdeckelementes den Signalleiter und damit das abgeleitete Signal nicht beeinflusst, ist gemäß der Erfindung vorgesehen, dass die elektrisch leitende Seite des Abdeckelementes vom Signalleiter galvanisch getrennt ist. Für diese galvanische Trennung eignet sich insbesondere die elektrisch isolierende Trägerschicht eines als mindestens zweilagige Folie ausgebildeten Abdeckelementes bzw. das Abdeckelement selber, sodass bei einer Querschnittsbetrachtung der Abgrifflasche der Signalleiter und die elektrisch leitende Seite des Abdeckelementes voneinander vertikal beabstandet, vorzugsweise zumindest bereichsweise überschneidend, angeordnet sind.

Wie an sich bekannt, kann gemäß einem Ausführungsbeispiel der Erfindung der Signalleiter mit dem signalgebenden Subjekt über ein vorzugsweise in einer Aussparung der Klebeschicht anordenbares elektrisch leitendes Gel verbunden sein und die Abgrifflasche ein Abgriffelement, vorzugsweise einen genieteten Kugelkopf, aufweisen, das mit dem Signalleiter elektrisch in Kontakt steht und von der dem signalgebenden Subjekt abgewandten Seite der Elektrode zugänglich ist.

Gemäß einem weiteren Ausführungsbeispiel der Erfindung ist das Abdeckelement mit der Abgrifflasche verbunden, vorzugsweise verklebt wodurch sich eine besonders zuverlässige Abschirmung des Signalleiters ergibt, insbesondere dann, wenn sich die elektrisch leitende Seite des Abdeckelementes zumindest teilweise bis auf die Oberseite der oberen Deckschicht erstreckt.

Wenn das Abdeckelement bzw. die Trägerschicht des Abdeckelementes einstückig mit der oberen Deckschicht der Elektrode ausgebildet ist, wirkt sich das, wie bereits erwähnt, günstig auf die Herstellungskosten aus.

Um Rötungen an der Haut des Patienten, die durch die Klebeschicht hervorgerufen werden können, weitestgehend zu vermeiden, sieht ein weiteres Ausführungsbeispiel der Erfindung vor, auf der dem signalgebenden Subjekt zugewandten Seite der Klebeschicht ein hautfreundliches, elektrisch isolierendes Pflasterelement anzuordnen. Ein einfacher Aufbau der erfindungsgemäßen Elektrode ergibt sich dabei, wenn das Abdeckelement bzw. die elektrisch isolierende Trägerschicht des Abdeckelementes vom Pflasterelement gebildet ist. Es wird also die der Abgrifflasche zugewandte Seite des die Abdecklasche überdeckenden Teiles des Pflasterelementes elektrisch leitend ausgebildet. Dabei kann die elektrische Seite wiederum als gitterförmiges, elektrisch leitendes Element, als metallisches Band, als elektrisch leitfähiger Schaum oder dergleichen ausgebildet sein. Ebenso kann ein als zweilagige Folie ausgebildetes Abdeckelement in einfacher Weise auf dem die Abgrifflasche überdeckenden Teil des Pflasterelementes aufgeklebt werden. Um bei diesem Ausführungsbeispiel der Erfindung zu verhindern, dass der Signalleiter mit der elektrisch leitenden Seite des auf dem Pflasterelement angeordneten Abdeckelementes in Kontakt gerät, kann, wie an sich bekannt, die dem signalgebenden Subjekt zugewandte Seite der Abgrifflasche mit einer Abdeckung aus Kunststoff versehen sein.

Eine weitere Ausführungsvariante der Erfindung sieht bei medizinischen Elektroden für die Ableitung von Signalen, insbesondere zur Signalableitung von der menschlichen Haut, mit einer Deckschicht, einer Klebeschicht zum Aufkleben der Elektrode auf die Haut und einem vorzugsweise metallischen, mit dem signalgebenden Subjekt verbindbaren Signalleiter, vor, zumindest die dem signalgebenden Subjekt abgewandte Seite der Deckschicht zumindest bereichsweise elektrisch leitend auszubilden. Eine besonders zuverlässige Abschirmung des Signalleiters gegenüber Störsignalen ergibt sich dann, wenn auch die Unterseite der Deckschicht zumindest bereichsweise elektrisch leitend ausgebildet ist. Wie schon bei den vorbeschriebenen Ausführungsbeispielen kann (können) die elektrisch leitende(n) Seite(n) der Deckschicht von einem gitterförmig ausgebildeten, elektrisch leitenden Element, einem elektrisch leitfähigen Schaum, einem metallischen Band, einem textilen Flächengebilde mit integrierten metallischen Fäden oder einer aufgedruckten elektrisch leitenden Schicht gebildet sein (werden).

Auch die Ausbildung der Deckschicht oder der elektrisch leitenden Seite als vorzugsweise zweilagige Folie mit einer ersten elektrisch isolierenden Trägerschicht und einer zweiten elektrisch leitenden Schicht ist denkbar. Unabhängig davon, wie die elektrisch leitende(n) Seite(n) der Deckschicht ausgebildet ist (sind), sieht ein weiteres Ausführungsbeispiel der Erfindung vor, dass die elektrisch leitende Seite der Deckschicht vom Signalleiter galvanisch getrennt ist, und gegebenenfalls vorhandene elektrisch leitende gesonderte Bereiche der Deckschicht vorzugsweise untereinander elektrisch verbunden sind, um eine Beeinflussung des Signalleiters durch die elektrisch leitende Seite der Deckschicht zu verhindern.
Ein weiteres Ausführungsbeispiel der Erfindung, bei dem die Elektrode eine Abgrifflasche aufweist, wobei auf der dem signalgebenden Subjekt zugewandten Seite der Abgrifflasche ein vorzugsweise elektrisch isolierendes Abdeckelement angeordnet, vorzugsweise geklebt, ist, sieht vor, das Abdeckelement einstückig mit der Deckschicht auszubilden. Dadurch kann das einstückig mit der Deckschicht hergestellte Abdeckelement und somit auch die Deckschicht aus statisch ungünstigen Kunststoffen, wie beispielsweise PE oder PET hergestellt sein, da eine statische Aufladung der Abdeckung durch die elektrisch leitende Seite der Deckschicht beinahe zur Gänze verhindert wird.

Um eine derartige statische Aufladung sicher verhindern zu können, sieht ein besonders bevorzugtes Ausführungsbeispiel der Erfindung vor, dass wenigstens eine Seite des vorzugsweise flächigen Abdeckelementes elektrisch leitend ausgebildet ist, wobei die elektrisch leitende Seite des Abdeckelementes vom Signalleiter galvanisch getrennt ist.

Sind die elektrisch leitende Seite der Deckschicht, der Signalleiter und die elektrisch leitende Seite des Abdeckelementes voneinander vertikal beabstandet, vorzugsweise zumindest bereichsweise überschneidend, angeordnet, ergibt sich durch diese Anordnung ein Effekt, der im wesentlichen dem Effekt eines Faraday'schen Schildes entspricht, da die vom Signalleiter abgewandten Seiten der Deckschicht und des Abdeckelementes elektrisch leitend ausgebildet sind und auf diese Weise die dem Signalleiter zugewandten Seiten der Deckschicht und des Abdeckelementes annähernd frei von Störsignalen halten.

Ein von äußeren Störeinflüssen beinahe zur Gänze abgeschirmter Signalleiter kann gemäß einem weiteren Ausführungsbeispiel auch dann erreicht werden, wenn auf der dem signalgebenden Subjekt zugewandten Seite der Klebeschicht ein hautfreundliches, elektrisch isolierendes Pflasterelement angeordnet ist, das die Abgrifflasche zumindest teilweise überdeckt, wobei der die Abgrifflasche überdeckende Teil des Pflasterelementes auf seiner der Abgrifflasche zugewandten Seite elektrisch leitend ausgebildet ist. Diese Ausführung wird vor allem bei der nachträglichen Applikation einer Abdeckfolie mit einer elektrisch leitfähigen Seite auf bereits auf dem Markt befindliche medizinische Elektroden und zwar sowohl auf die Deckseite als eben auch auf dem die Abgrifflasche überdeckenden Teil des Pflasterelementes zur Anwendung gelangen.

Eine weitere Ausführungsvariante der Erfindung sieht vor, eine medizinische Elektrode für die Ableitung von Signalen, insbesondere zur Signalableitung von der menschlichen Haut, mit einer Deckschicht, einer Klebeschicht zum Aufkleben der Elektrode auf die Haut und einem vorzugsweise metallischen, mit dem signalgebenden Subjekt verbindbaren Signalleiter, die vorzugsweise eine Abgrifflasche aufweist, mit mindestens einer zusätzlichen, vorzugsweise in Form eines Faraday'schen Schildes bzw. Bechers angeordneten, elektrisch leitenden Schicht auszustatten, die den Signalleiter gegenüber Störsignalen abschirmt.

Zum Ableiten der Störsignale von bzw. zum Erden der elektrisch leitenden Schicht, die vorzugsweise vom Signalleiter galvanisch getrennt ist, sieht die Erfindung vor, dass die elektrisch leitende Schicht mit einem Leiter zum Ableiten der elektrischen Störspannung bzw. störender elektrischer Aktivität oder störender elektrischer Potentiale in Kontakt bringbar ausgebildet ist. Günstigerweise weist die elektrisch leitende Schicht zu diesem Zweck ein Anschlusselement zum lösbaren Befestigen des Leiters auf.

Eine besonders einfache Handhabung ergibt sich, wenn die Übertragung der Signale des Signalleiters und der Störsignale der elektrisch leitenden Schicht über eine Leitung, die zwei galvanisch getrennte Leiter aufweist, erfolgt, wodurch erreicht wird, dass von jeder Elektrode lediglich ein Kabel zur Auswerteeinheit führt, was besonders bei Messungen während sportlicher Aktivitäten von großem Vorteil ist.

Günstigerweise wird die Leitung dabei von einem Koaxialkabel gebildet, bei dem die beiden galvanisch getrennten Leiter der Leitung koaxial angeordnet bzw. ausgebildet sind, wobei der innere Leiter mit dem Signalleiter in Kontakt bringbar ist, während der äußere Leiter des Kabels mit der elektrisch leitenden Schicht in Kontakt steht. Mit dieser Maßnahme kann verhindert werden, dass Bewegungen im bzw. des Kontaktbereichs der Leitung zu Störungen des Messsignals führen können, da auftretende elektrische Störspannungen, elektrische Aktivitäten bzw. elektrische Potentiale über die elektrische leitende Schicht und den äußeren Leiter der Leitung abgeleitet werden. Das bedeutet, dass die Relativbewegungen der Leitung bzw. der Kontaktelemente der Leitung zur Elektrode, die nach dem bisherigen Stand der Technik das abgeleitete Signal beeinflusst haben, bei einer erfindungsgemäßen Elektrode auf das abgeleitete Signal beinahe keine Auswirkung mehr haben bzw. zur Gänze vernachlässigt werden können.

Die Leitung kann mit der Elektrode schnell und einfach in Kontakt gebracht werden, wenn die Leitung ein Anschlusselement aufweist, mittels dem die elektrisch leitende Schicht und der Signalleiter gleichzeitig mit ihren korrespondierenden Leitern der Leitung in Kontakt bringbar sind, wie ein weiteres Ausführungsbeispiel vorsieht.

Weitere Vorteile und Einzelheiten werden anhand der nachfolgenden Beschreibung unter Bezugnahme auf die in den Zeichnungen dargestellten Figuren näher erläutert. Darin zeigen die
- Fig. 1a bis 1c: eine medizinische Elektrode nach dem Stand der Technik ohne Abdeckelement für die Angrifflasche,
- Fig. 2a bis 2c: eine medizinische Elektrode nach dem Stand der Technik mit einem Abdeckelement für die Abgrifflasche,
- Fig. 3a bis 3c: ein erstes Ausführungsbeispiel der Erfindung,
- Fig. 4a bis 4c: ein weiteres Ausführungsbeispiel der Erfindung,
- Fig. 5a: eine erfindungsgemäße Abdeckung,
- Fig. 5b und 5c: unterschiedliche Anordnungsmöglichkeiten der in Fig. 5a dargestellten Abdeckung an einer medizinischen Elektrode,
- Fig. 6a bis 6c: sowie
- Fig. 7a bis 7c: weitere Ausführungsvarianten einer erfindungsgemäßen Elektrode mit einer Abgrifflasche,
- Fig. 8a und 8b: schematisch zwei Beispiele einer Elektrode ohne Abgrifflasche,
- Fig. 9a und 9b: schematisch unterschiedliche Befestigungsmöglichkeiten des Leiters für das Störsignal mit der elektrischen Schicht und
- Fig. 10 und 11: unterschiedliche Verbindungsmöglichkeiten der Leitung zum Ableiten der Signale mit der Elektrode.

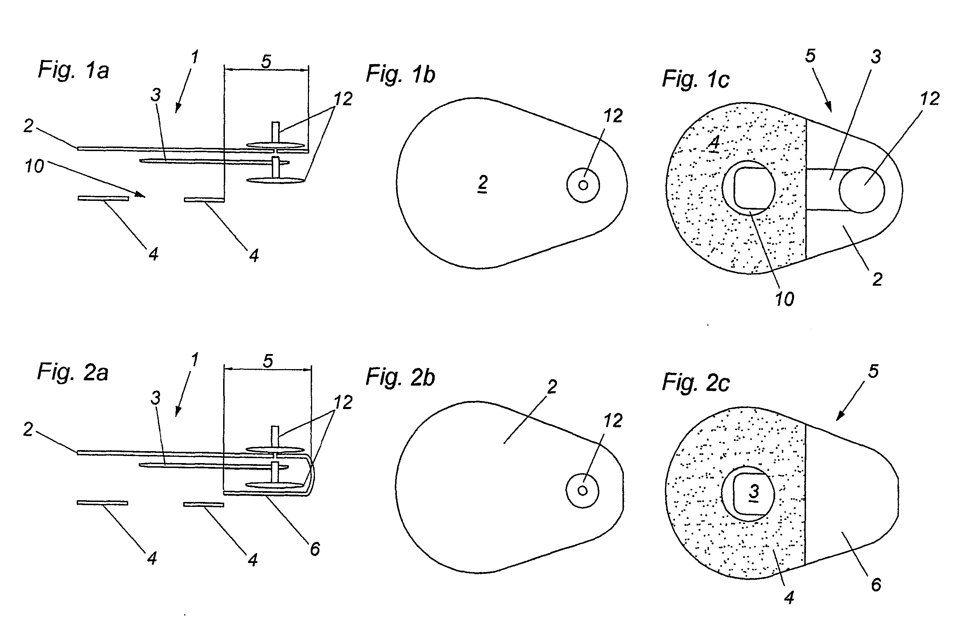

Die in den Figuren 1a bis 1c dargestellte medizinische Elektrode 1 nach dem Stand der Technik ist dreischichtig aufgebaut und weist eine Deckschicht 2, die von der Haut des Patienten abgewandt ist, und eine Klebeschicht 4, mit der die Elektrode 1 auf der Haut des Patienten befestigt wird, auf. Zwischen diesen beiden Schichten 2, 4 ist ein Signalleiter 3 angeordnet. Der Signalleiter 3 steht mit der Haut des Patienten beispielsweise über ein Leitgel (nicht dargestellt), das in einer Aussparung 10 der Klebeschicht 4 angeordnet ist, in Kontakt. Die Elektrode 1 weist eine Abgrifflasche 5 auf, die von einem Abschnitt der Deckschicht 2 und einem Abschnitt des Signalleiters 3 gebildet wird und ein Abgriffelement 12, beispielsweise in Form eines zweiteiligen zusammengenieteten Druckknopfes, dessen Oberteil als Kugelkopf ausgebildet sein kann, auf. Alternativ wäre auch die Ausbildung der Angrifflasche 5 zum Anschluss eines elektrischen Drahtes mit einer Krokodil-Klemme möglich.

Auch die Anordnung eines Pflasterelementes (nicht dargestellt), auf der der Haut des Patienten zugewandten Seite der Klebeschicht 4 ist möglich. In diesem Fall ist es günstig, das Pflasterelement derart auszubilden, dass es die Abgrifflasche 5 überdeckt und auf diese Weise einen Kontakt des Signalleiters 3 mit der Haut des Patienten verhindert.

Fig. 1b zeigt die Draufsicht auf die Elektrode 1, bei der nur die Deckschicht 2 und der Oberteil des Druckknopfes 12 sichtbar ist. Betrachtet man die Elektrode, wie in Fig. 1c dargestellt, von der Unterseite, erkennt man, dass die Deckschicht 2, außer im Bereich der Abgrifflasche 5, bis auf die Aussparung 10 von der Klebeschicht 4 bedeckt ist. Die Aussparung 10 dient der Anordnung eines Leitgels (nicht dargestellt), das den Kontakt zwischen der Haut des Patienten mit dem Signalleiter 3 durch die Klebeschicht 4 hindurch herstellt. Im Bereich der Abgrifflasche 5 ist auf der Unterseite der oberen Deckschicht 2 der Signalleiter 3, über den der Unterteil des genieteten Druckknopfes 12 mit der Haut des Patienten in Verbindung steht, angeordnet. Bei einer derartig ausgebildeten Elektrode 1 ist der Signalleiter 3 äußeren Störsignalen ungeschützt ausgesetzt.

Eine medizinische Elektrode 1, bei der der Signalleiter 3 mittels einer Abdeckung 6 gegenüber äußeren Störeinflüssen geschützt ist, geht aus den Fig. 2a bis 2c hervor. Dabei ist die Abdeckung 6 einstückig mit der Deckschicht 2 hergestellt und wird von dieser gebildet. Wie bereits erwähnt, ladet sich bei derart ausgebildeten Elektroden 1 die Abdeckung 6 bzw. die Deckschicht 2 statisch auf und beeinflusst, da sie ja mit dem Signalleiter 3 in Kontakt steht, das von der Haut des Patienten über den Signalleiter 3 und das Abgriffelement 12 der Abgrifflasche 5 abgeleitete Signal, sodass die erzielten Messergebnisse nicht zuverlässig sind. Im Gegensatz zur in Fig. 1c dargestellten medizinischen Elektrode ist aus Fig. 2c, die die Unteransicht der in Fig. 2a dargestellten medizinischen Elektrode 1 zeigt, ersichtlich, dass die Abgrifflasche 5 auf ihrer der Haut des Patienten zugewandten Seite mit einer Abdeckung 6 versehen ist. Diese Abdeckung 6 kann, wie dargestellt, einstückig mit der oberen Deckschicht 2 ausgebildet sein, aber auch die Anordnung einer separaten Abdeckung 6 ist möglich.

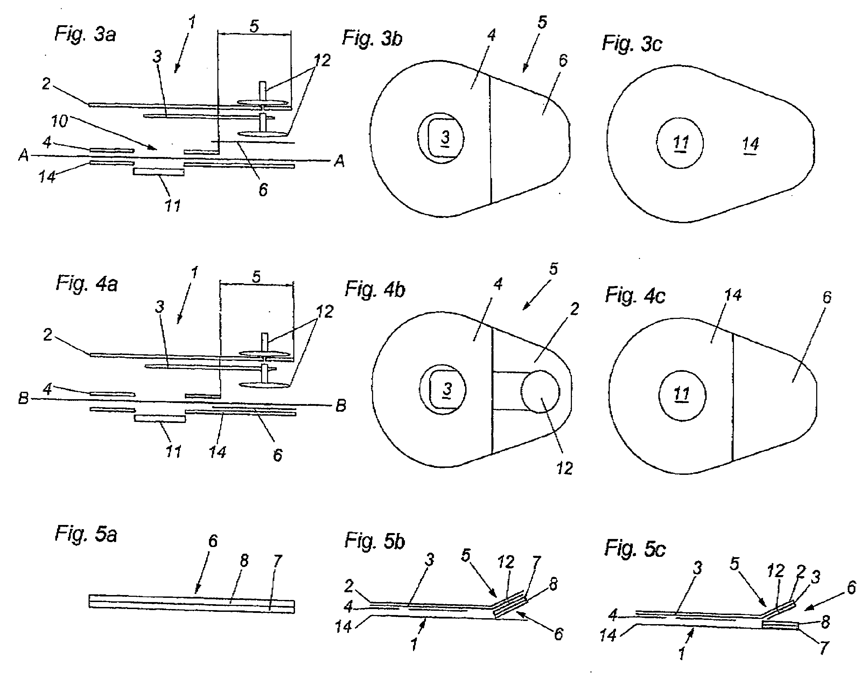

In den Fig. 3a bis 3c ist ein erstes Ausführungsbeispiel einer erfindungsgemäßen medizinischen Elektrode 1 dargestellt. Dabei ist unterhalb der Klebeschicht 4 auf der der Haut des Patienten zugewandten Seite der Elektrode 1 ein Pflasterelement 14 angeordnet, das eine mit der Aussparung 10 der Klebeschicht 4 annähernd deckungsgleiche Durchtrittsöffnung, in der ein elektrisch leitendes Gel 11, das den Kontakt zwischen der Haut des Patienten und dem Signalleiter 3 herstellt, angeordnet ist, aufweist. Auf der der Haut zugewandten Seite der Abdecklasche 5 ist eine Abdeckung 6 angeordnet, deren eine Seite elektrisch leitend ausgebildet ist. Diese Abdeckung 6 deckt wie in Fig. 3b, die eine Untersicht des oberen Teiles der medizinischen Elektrode 1 entlang des Schnittes A-A darstellt, gezeigt die Abgrifflasche 5 zur Gänze ab. Die in Fig. 3b sichtbare Seite der Abdeckung 6 ist elektrisch leitend ausgebildet, wobei die elektrische Leitfähigkeit beispielsweise durch Aufbringung eines elektrisch leitenden Schaumes bzw. durch Aufdrucken einer elektrisch leitenden Schicht erzielt wird.

Wenn auch eine optimale elektrische Leitfähigkeit durch die Ausbildung der Abdeckung 6, wie sie in Fig. 3b dargestellt ist, erreicht wird, kann ein gewisser Abschirmeffekt auch dann erreicht werden, wenn die Abdeckung 6 lediglich den auf der Abdecklasche 5 angeordneten Teil des Signalleiters 3 überdeckend ausgebildet ist. Fig. 3c zeigt eine Draufsicht auf das Pflasterelement 14 entlang der Schnittlinie A-A, wobei in der Durchtrittsöffnung das elektrisch leitende Gel 11 angeordnet ist.

Beim Ausführungsbeispiel nach den Fig. 4a bis 4c ist die Abdeckung 6, deren eine Seite elektrisch leitend ausgebildet oder die durchgehend leitfähig ist, nicht an der Abdecklasche 5, sondern auf dem die Abdecklasche überdeckenden Teil des Pflasterelementes 14 angeordnet. Bei einer derartigen Anordnung des Abdeckelementes 6 ist darauf zu achten, dass die elektrisch leitende Seite des Abdeckelementes 6 der Abgrifflasche 5 zugewandt ist. Um einen Kontakt des Abdeckelementes 6 mit dem Signalleiter 3 bzw. dem Unterteil des Abgriffelementes 12 zu vermeiden, kann die dem Pflasterelement 14 zugewandte Seite der Abgrifflasche 5 zusätzlich mit einem elektrisch isolierenden Abdeckelement (nicht dargestellt) versehen sein. Analog zur Fig. 3b zeigt die Fig. 4b die Unteransicht der medizinischen Elektrode 1 entlang der Schnittlinie B-B, aus der ersichtlich ist, dass im Bereich der Abgrifflasche 5 auf der Unterseite der oberen Deckschicht 2 der Signalleiter 3 angeordnet ist. Die Fig. 4c zeigt die Obersicht auf das Pflasterelement 14 entlang der Schnittlinie B-B mit einer auf dem die Abgrifflasche überdeckenden Teil des Pflasterelementes 14 angeordneten Abdeckelement 6.

Eine bevorzugte Ausführungsform der erfindungsgemäßen Abdeckung ist in der Fig. 5a dargestellt. Dabei ist das Abdeckelement 6 als zweilagige Folie ausgebildet und weist eine elektrisch isolierende Trägerschicht 7 sowie eine elektrisch leitende Schicht 8 auf. Diese elektrisch leitende Schicht 8 kann beispielsweise von einem gitterförmig ausgebildeten, elektrisch leitenden Element gebildet oder aufgedruckt werden. Ebenso zweckmäßig ist die Ausbildung der elektrisch leitenden Schicht 8 mittels eines elektrisch leitenden Schaumes, eines metallischen Bandes, eines textilen Flächengebildes mit integrierten metallischen Fäden oder dergleichen. In den Fig. 5b und 5c sind zwei unterschiedliche Anordnungsmöglichkeiten einer Abdeckung 6 nach Fig. 5a an einer medizinischen Elektrode 1 dargestellt. Bei der in 5b dargestellten Variante ist die Abdeckung 6 an der Abgrifflasche 5, und zwar auf der der Haut des Patienten zugewandten Seite angeordnet. Dabei ist die Abdeckung 6 mit ihrer elektrisch isolierenden Trägerschicht 7 mit der Abgrifflasche 5 verbunden, sodass die elektrisch leitende Schicht 8 vom Signalleiter 3 galvanisch getrennt ist. Bei der in Fig. 5c gezeigten Variante, ist das Abdeckelement 6 mit dem Pflasterelement 14 verbunden, und zwar derart, dass die elektrisch leitende Schicht 8 der Abgrifflasche 5 zugewandt ist. Um zu verhindern, dass die elektrische Schicht 8 der Abdeckung 6 mit dem Signalleiter 3 in Kontakt gerät, kann die Abgrifflasche 5 auf der der Abdeckung 6 zugewandten Seite mit einer elektrisch isolierenden Abdeckung versehen sein, die beispielsweise einstückig mit der oberen Deckschicht ausgebildet sein kann.

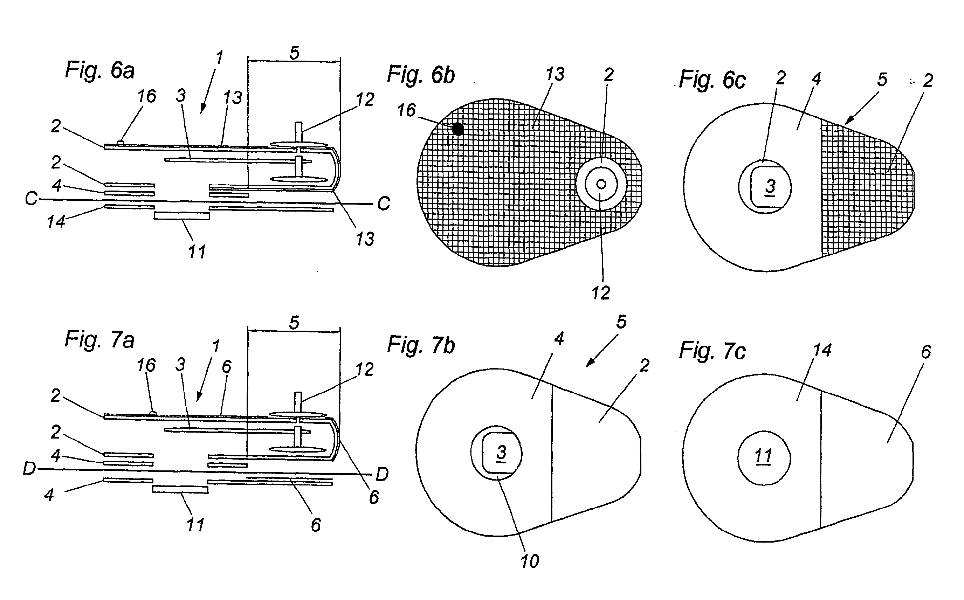

Bei den in den Fig. 6a bis 6c und 7a bis 7c dargestellten Ausführungsvarianten der Erfindung ist zumindest die von der Haut des Patienten abgewandte Seite 13 der Deckschicht 2 elektrisch leitend ausgebildet.

Beim Ausführungsbeispiel nach den Fig. 6a bis 6c weist die Abgrifflasche 5 auf ihrer der Haut zugewandten Seite zusätzlich eine Abdeckung auf, die einstückig mit der oberen Deckschicht 2 hergestellt und von dieser gebildet wird. Dadurch wird einerseits die Elektrode zur Gänze bzw. dreidimensional und andererseits die der Haut zugewandte Seite der Abgrifflasche 5 im speziellen von einer elektrisch leitenden Schicht 13 bedeckt, sodass äußere Störsignale vom Signalleiter 3 abgehalten werden können. Fig. 6b zeigt eine Draufsicht auf die in Fig. 6a dargestellt medizinische Elektrode 1, aus der hervorgeht, dass die elektrisch leitende Schicht 13 gitterförmig ausgebildet ist. Das elektrisch leitende Gitter 13 reicht bis knapp vor den Oberteil des Abgriffelementes 12, steht mit diesem jedoch nicht in Berührung, sodass zwischen dem Abgriffelement 12 und dem Gitter 13 die Deckschicht 2 sichtbar ist. Aus Fig. 6c, die eine Untersicht der medizinischen Elektrode 1 entlang der Schnittlinie C-C darstellt, ist ersichtlich, dass die Deckschicht 2 im Bereich der Abgrifflasche 5 vom elektrisch leitenden Gitter 13 bedeckt ist, während der Rest der Deckschicht 2 bis auf die Aussparung für das elektrisch leitende Gel 11 von der Klebeschicht 4 bedeckt ist. Das Anschlusselement 16 stellt die Masse dar und dient zur Erdung der elektrisch leitenden Schicht 13. Selbstverständlich wäre es auch denkbar, die der Haut zugewandten Seite der Abgrifflasche 5 anstelle der mit einer elektrisch leitenden Seite 13 ausgestatteten Deckschicht 2 mit einem separat aufzubringenden Abdeckelement 6 zu versehen.

Ist bei dem in den Fig. 6a bis 6c gezeigten Ausführungsbeispiel die Deckschicht als zweilagige Folie mit einer elektrisch isolierenden Deckschicht 2 und einer elektrisch leitenden Schicht 13 ausgebildet, weist im Gegensatz dazu bei den in Fig. 7a bis 7c gezeigten Ausführungsbeispiel die obere Deckschicht auf ihrer der Haut des Patienten abgewandten Seite ein Abdeckelement 6 mit wenigstens einer elektrisch leitenden Seite auf. Vorteilhafterweise ist das Abdeckelement 6 zweilagig ausgebildet, wobei die Anordnung des Abdeckelementes 6 derart erfolgt, dass die elektrisch leitende Seite von der Deckschicht 2 abgewandt ist. Die Deckschicht 2 ist im Bereich der Abgrifflasche 5 umgeschlagen und deckt damit den Signalleiter 3 zumindest im Bereich der Abgrifflasche 5 gegenüber äußeren Einflüssen ab. Durch die Anordnung der Abdeckung 6 auch entlang der Seitenfläche der Abgrifflasche 5 kann eine statische Aufladung der auf der Unterseite der Abgrifflasche 5 angeordneten Deckschicht 2, die im Normalfall aus Kunststoff hergestellt ist, beinahe zur Gänze vermieden werden. Zusätzlich ist beim gezeigten Ausführungsbeispiel auf dem die Abgrifflasche 5 überdeckenden Teil des Pflasterelementes 14 eine Abdeckung 6 derart angeordnet, dass die elektrisch leitende Seite der Abdeckung 6 der Abgrifflasche 5 zugewandt ist.

Aus der Fig. 7b, die eine Untersicht der Elektrode 1 nach Schnitt D-D zeigt, ist ersichtlich, dass die Abgrifflasche 5 auf ihrer der Haut des Patienten zugewandten Seite von der Deckschicht 2 abgedeckt ist, während der restliche Teil der Elektrode 1 mit einer Klebeschicht 4 versehen ist. Die Aussparung 10 in der Klebeschicht 4 dient wiederum der Aufnahme eines elektrisch leitfähigen Gels 11, mittels dem der Signalleiter 3 mit der Haut des Patienten in Verbindung steht. Fig. 7c zeigt eine Draufsicht auf das Pflasterelement 14 entlang der Schnittlinie D-D, aus der hervorgeht, dass der die Abgrifflasche 5 überdeckende Teil des Pflasterelementes 14 mit einer Abdeckung 6 versehen ist, wobei die elektrisch leitende Seite des Abdeckelementes 6 der Abgrifflasche 5 zugewandt ist. In einer Durchtrittsöffnung des Pflasterelementes 14, die im wesentlichen der Aussparung 10 in der Klebeschicht 4 entspricht, ist das Leitgel 11 angeordnet.

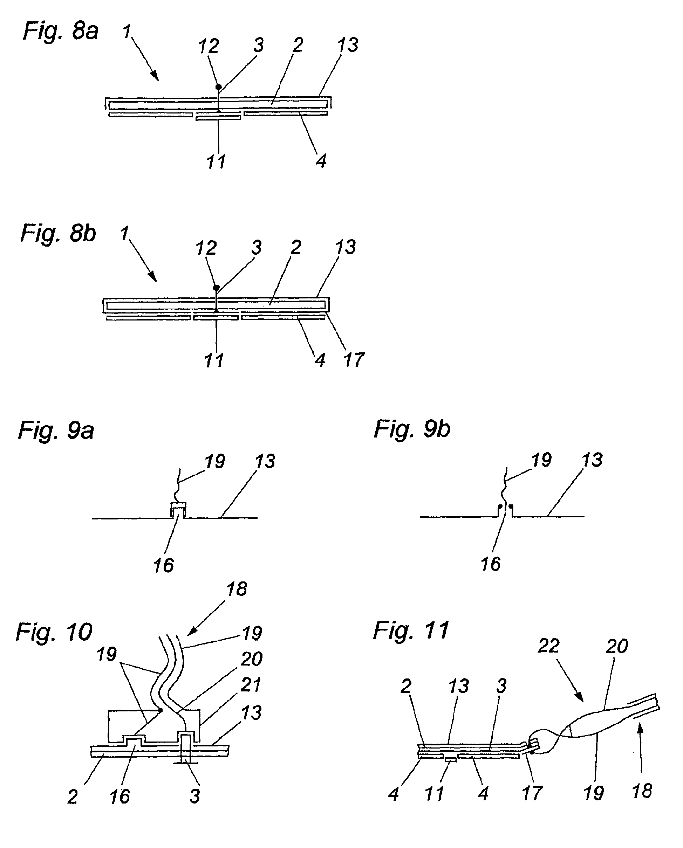

Fig. 8a und 8b zeigen jeweils eine medizinische Elektrode 1 ohne Abgrifflasche, das heißt, das Abgriffelement 12 liegt direkt über dem Bereich des Signalleiters 3, in dem dieser mit dem signalgebenden Subjekt, vorzugsweise über ein leitfähiges Gel 11, in Kontakt steht. Die Deckschicht 2, die im Wesentlichen den Träger der Elektrode 1 bildet, weist auf Ihrer, der Haut des Patienten abgewandten Seite, eine elektrische leitende Schicht 13 auf. Auf der der Haut zugewandten Seit der Deckschicht 2 ist zumindest bereichsweise eine Klebeschicht 4 zum Aufkleben der Elektrode 1 auf die Haut angeordnet. Die elektrisch leitende Schicht 13 verfügt über eine ableitende Wirkung, das heißt, der Aufbau einer statischen Aufladung, die das abgeleitete Signal beeinflussen könnten, wird verhindert. In Fig. 8b ist auf der der Haut des Patienten zugewandten Seite der Deckschicht 2, zwischen der Deckschicht 2 und der Klebeschicht 4, ebenfalls eine elektrisch leitende Schicht 17 angeordnet, sodaß die Deckschicht 2 allseitig, das heißt dreidimensional, von einer elektrisch leitenden Schicht 13, 17 zur Ableitung von Störsignalen umschlossen ist.

In den Fig. 9a und 9b sind zwei unterschiedliche Möglichkeiten einer Steckverbindung zwischen dem Anschlusselement 16 der elektrisch leitenden Schicht 13 und dem Leiter 19 zum Ableiten der Störsignale dargestellt. Nach Fig. 9a ist das Anschlusselement 16 beispielsweise in Art eines Druckknopfes ausgebildet. Der Leiter 19 ist an seinem Ende kappenförmig ausgebildet und wird auf das Anschlusselement 16 aufgesteckt. Im Gegensatz dazu, weist das Anschlusselement 16 nach Fig. 9b eine Öffnung auf, in welcher der Leiter 19 eingesteckt wird. Das Anschlusselement 16 kann dabei federnd ausgebildet sein, sodaß der Leiter 19 klemmschlüssig gehalten wird.

Bei dem in Fig. 10 dargestellten Ausführungsbeispiel ist die Leitung 18 als Koaxialkabel ausgebildet und weist ein Anschlusselement 21 auf, mit dem das Anschlusselement 16 der elektrisch leitenden Schicht 13 und der Signalleiter 3 gleichzeitig kontaktiert werden können. Dabei bildet der äußere der beiden Leiter 19 die Abschirmung für den mit dem Signalleiter 3 verbundenen Leiter 20 und gleichzeitig das Gehäuse des Anschlusselementes 21 bzw. ist mit diesem elektrisch verbunden. Auf diese Weise stellt der Leiter 19 die Erdung für die elektrisch leitende Schicht 13 dar. Der Leiter 20, mittels dem Messsignal vom Signalleiter 3 abgeleitet wird, ist vom Leiter 19 bzw. vom Gehäuse 21 und damit von der elektrisch leitenden Schicht 13 galvanisch getrennt.

Beim Ausführungsbeispiel nach Fig. 11 erfolgt die Abnahme sowohl des Störsignals als auch des Messsignals über Klemmvorrichtung 22, beispielsweise eine Krokodilklemme 22. Dabei greift ein Klemmenteil 19 an der elektrisch leitenden Schicht 17, die der Abschirmung des Signalleiters 3 dient, an, während der zweite Klemmenteil 20, der vom ersten Klemmenteil 19 galvanisch getrennt ist, direkt am Signalleiter 3 angreift und auf diese Weise das Messsignal weiterleitet.

Wenn auch die Erfindung anhand der gezeigten Ausführungsbeispiele detailliert beschrieben wurde, versteht es sich von selbst, dass die Erfindung nicht auf die gezeigten Ausführungsbeispiele beschränkt ist. Vielmehr werden alle möglichen Kombinationen der in den Ansprüchen beschriebenen Ausführungsbeispiele, die dazu geeignet sind, eine grundliegende Idee der Erfindung, nämlich die Schaffung einer medizinischen Elektrode, bei der der eigentliche Signalleiter mittels einer zusätzlichen elektrisch leitenden Schicht, nach dem Prinzip eines Faraday'schen Schildes bzw. Bechers, von Störsignalen freigehalten wird, umzusetzen, beansprucht.

## Patentansprüche

1. Medizinische Elektrode (1) für die Ableitung von Signalen, insbesondere zur Signalableitung von der menschlichen Haut, die wenigstens zweischichtig aufgebaut ist, wobei zwischen einer oberen Deckschicht (2) und einer mit dem signalgebenden Subjekt kontaktierbaren Klebeschicht (4) ein vorzugsweise metallischer, mit dem signalgebenden Subjekt verbindbarer Signalleiter (3), vorzugsweise in Form einer Signalleitschicht, angeordnet ist, und die eine Abgrifflasche (5) aufweist, wobei zwischen der Abgrifflasche (5) und dem signalgebenden Subjekt ein elektrisch isolierendes Abdeckelement (6) angeordnet ist, **dadurch gekennzeichnet, dass** wenigstens eine Seite des vorzugsweise flächigen Abdeckelementes (6) elektrisch leitend ausgebildet und vom Signalleiter (3) galvanisch getrennt ist, wobei die wenigstens eine elektrisch leitende Schicht (13, 17, 8) mit einem Leiter (19) zum Ableiten der Störsignale bzw. zum Erden in Kontakt bringbar ausgebildet ist.

2. Elektrode nach Anspruch 1, **dadurch gekennzeichnet, dass** die elektrisch leitende Seite des Abdeckelementes (6) von einem gitterförmig ausgebildeten, elektrisch leitenden Element (8) gebildet ist.

3. Elektrode nach Anspruch 1, **dadurch gekennzeichnet, dass** die elektrisch leitende Seite des Abdeckelementes (6) von einem elektrisch leitfähigen Schaum (8) gebildet ist.

4. Elektrode nach einem der Ansprüche 1 bis 3, **dadurch gekennzeichnet, dass** das Abdeckelement (6) als vorzugsweise zweilagige Folie mit einer ersten elektrisch isolierenden Trägerschicht (7) und einer zweiten elektrisch leitenden Schicht (8) ausgebildet ist.

5. Elektrode nach einem der Ansprüche 1 bis 4, **dadurch gekennzeichnet, dass** der Signalleiter (3) und die elektrisch leitende Seite des Abdeckelementes (6) voneinander vertikal beabstandet, vorzugsweise zumindest bereichsweise überschneidend, angeordnet sind.

6. Elektrode nach einem der Ansprüche 1 bis 5, **dadurch gekennzeichnet, dass** der Signalleiter (3) mit dem signalgebenden Subjekt über ein vorzugsweise in einer Aussparung (10) der Klebeschicht (4) anordenbares, elektrisch leitendes Gel (11) verbunden ist.

7. Elektrode nach einem der Ansprüche 1 bis 6, **dadurch gekennzeichnet, dass** die Abgrifflasche (5) ein Abgriffelement (12), vorzugsweise einen genieteten Kugelkopf, aufweist, das mit dem Signalleiter (3) elektrisch in Kontakt steht und von der dem signalgebenden Subjekt abgewandten Seite der Elektrode (1) zugänglich ist.

8. Elektrode nach einem der Ansprüche 1 bis 7, **dadurch gekennzeichnet, dass** das Abdeckelement (6) mit der Abgrifflasche (5) verbunden, vorzugsweise verklebt, ist.

9. Elektrode nach einem der Ansprüche 1 bis 8, **dadurch gekennzeichnet, dass** das Abdeckelement (6) bzw. die Trägerschicht (7) des Abdeckelementes (6) einstückig mit der oberen Deckschicht (2) der Elektrode (1) ausgebildet ist.

10. Elektrode nach einem der Ansprüche 1 bis 9, **dadurch gekennzeichnet, dass** zumindest die dem signalgebenden Subjekt abgewandte Seite (13) der Deckschicht (2) zumindest bereichsweise elektrisch leitend ausgebildet ist.

11. Elektrode nach Anspruch 10, **dadurch gekennzeichnet, dass** auch die Unterseite (17) der Deckschicht (2) zumindest bereichsweise elektrisch leitend ausgebildet ist.

12. Elektrode nach Anspruch 10 oder 11, **dadurch gekennzeichnet, dass** die elektrisch leitende Seite (13, 17) der Deckschicht (2) von einem gitterförmig ausgebildeten, elektrisch leitenden Element gebildet ist.

13. Elektrode nach Anspruch 10 oder 11, **dadurch gekennzeichnet, dass** die elektrisch leitende Seite (13, 17) der Deckschicht (2) von einem elektrisch leitfähigen Schaum gebildet ist.

14. Elektrode nach Anspruch 10 bis 13, **dadurch gekennzeichnet, dass** die elektrisch leitende Schicht (13, 17) von einer aufgedruckten, elektrisch leitenden Schicht gebildet ist.

15. Elektrode nach einem der Ansprüche 10 bis 14, **dadurch gekennzeichnet, dass** die Deckschicht (2) als vorzugsweise zweilagige Folie mit einer ersten elektrisch isolierenden Trägerschicht (7) und einer zweiten elektrisch leitenden Schicht (8) ausgebildet ist.

16. Elektrode nach einem der Ansprüche 10 bis 14, **dadurch gekennzeichnet, dass** die elektrisch leitende Seite (13, 17) als vorzugsweise zweilagige Folie mit einer ersten elektrisch isolierenden Trägerschicht (7) und einer zweiten elektrisch leitenden Schicht (8) ausgebildet ist.

17. Elektrode nach einem der Ansprüche 10 bis 16, **dadurch gekennzeichnet, dass** die elektrisch leitende Seite (13, 17) der Deckschicht (2) vom Signalleiter (3) galvanisch getrennt ist und gegebenenfalls vorhandene elektrisch leitende gesonderte Bereiche der Deckschicht vorzugsweise untereinander elektrisch verbunden sind.

18. Elektrode nach einem der Ansprüche 10 bis 17, **dadurch gekennzeichnet, dass** die elektrisch leitende Seite (13) der Deckschicht (2), der Signalleiter (3) und die elektrisch leitende Seite des Abdeckelementes (6, 17) voneinander vertikal beabstandet, vorzugsweise zumindest bereichsweise überschneidend, angeordnet sind.

19. Elektrode nach einem der Ansprüche 1 bis 18, **dadurch gekennzeichnet, dass** auf der dem signalgebenden Subjekt zugewandten Seite der Klebeschicht (4) ein hautfreundliches, elektrisch isolierendes Pflasterelement (14) angeordnet ist.

20. Elektrode nach Anspruch 19, **dadurch gekennzeichnet, dass** das Pflasterelement (14) die Abgrifflasche (5) zumindest teilweise überdeckt, wobei der die Abgrifflasche (5) überdeckende Teil des Pflasterelementes (14) auf seiner der Abgrifflasche (5) zugewandten Seite elektrisch leitend ausgebildet ist.

21. Elektrode nach Anspruch 19 oder 20, **dadurch gekennzeichnet, dass** das Abdeckelement (6) bzw. die elektrisch isolierende Trägerschicht (7) des Abdeckelementes (6) vom Pflasterelement (14) gebildet ist.

22. Elektrode nach einem der Ansprüche 1 bis 21, **dadurch gekennzeichnet, dass** die elektrisch leitende Schicht (13, 17, 8), die den Signalleiter (3) gegenüber Störsignalen abschirmt, in Form eines Faraday'schen Schildes bzw. Bechers angeordnet ist.

23. Elektrode nach einem der Ansprüche 1 bis 22, **gekennzeichnet durch** ein an der elektrisch leitenden Schicht angeordnetes bzw. mit dieser verbundenes Anschlusselement (16) zum lösbaren Befestigen des Leiters (19) an der elektrisch leitenden Schicht (13, 17, 8).

24. Elektrode nach einem der Ansprüche 1 bis 23, **dadurch gekennzeichnet, dass** die Übertragung der Signale des Signalleiters (3) und der Störsignale der elektrisch leitenden Schicht (13, 17, 8) über eine Leitung (18), die zwei galvanisch getrennte Leiter (19, 20) aufweist, erfolgt.

25. Elektrode nach Anspruch 24, **dadurch gekennzeichnet, dass** die beiden Leiter (19, 20) der Leitung (18) koaxial angeordnet bzw. ausgebildet sind, wobei der innere Leiter (20) mit dem Signalleiter (3) in Kontakt bringbar ist.

26. Elektrode nach einem der Ansprüche 1 bis 25, **dadurch gekennzeichnet, dass** die Leitung (18) ein Anschlusselement (21) aufweist, mittels dem die elektrisch leitende Schicht (13, 17, 8) und der Signalleiter (3) gleichzeitig mit ihren korrespondierenden Leitern (19, 20) der Leitung (18) in Kontakt bringbar sind.

## Claims

1. Medical electrode (1) for conducting signals, in particular for conducting signals from the human skin, which is built up in at least two layers, wherein a preferably metallic signal conductor (3), preferably in the form of a signal conducting layer, which can be connected to the signal-emitting subject is arranged between an upper top layer (2) and an adhesive layer (4) which can be brought into contact with the signal-emitting subject, and which has a tapping pad (5), an electrically insulating covering element (6) being arranged between the tapping pad (5) and the signal-emitting subject, **characterized in that** at least one side of the preferably planar covering element (6) is electrically conductive in construction and is galvanically separated from the signal conductor (3), wherein the at least one electrically conducting layer (13, 17, 8) is constructed such that it can be brought into contact with a conductor (19) for conducting the interference signals or for earthing.

2. Electrode according to claim 1, **characterized in that** the electrically conductive side of the covering element (6) is formed by an electrically conductive element (8) having a grid-like construction

3. Electrode according to claim 1, **characterized in that** the electrically conductive side of the covering element (6) is formed by an electrically conductive foam (8).

4. Electrode according to one of claims 1 to 3, **characterized in that** the covering element (6) is constructed as a preferably two-layered film having a first electrically insulating carrier layer (7) and a second electrically conductive layer (8).

5. Electrode according to one of claims 1 to 4, **characterized in that** the signal conductor (3) and the electrically conductive side of the covering element (6) are arranged with vertical spacing from one another, preferably overlapping at least in regions.

6. Electrode according to one of claims 1 to 5, **characterized in that** the signal conductor (3) is connected to the signal-emitting subject via an electrically conductive gel (11) which can preferably be arranged in an opening (10) of the adhesive layer (4).

7. Electrode according to one of claims 1 to 6, **characterized in that** the tapping pad (5) has a tapping element (12), preferably a riveted ball-head, which is in electrical contact with the signal conductor (3) and is accessible from the side of the electrode (1) facing away from the signal-emitting subject.

8. Electrode according to one of claims 1 to 7, **characterized in that** the covering element (6) connected to the tapping pad (5), preferably by adherence.

9. Electrode according to one of claims 1 to 8, **characterized in that** the covering element (6) or the carrier layer (7) of the covering element (6) is constructed in one piece with the upper top layer (2) of the electrode (1).

10. Electrode according to one of claims 1 to 9, **characterized in that** at least the side (13) of the top layer (2) facing away from the signal-emitting subject is electrically conductive in construction at least in regions.

11. Electrode according to claim 10, **characterized in that** the under-side (17) of the top layer (2) is also electrically conductive in construction at least in regions.

12. Electrode according to claim 10 or 11, **characterized in that** the electrically conductive side (13, 17) of the top layer (2) is formed by an electrically conductive element having a grid-like construction.

13. Electrode according to claim 10 or 11, **characterized in that** the electrically conductive side (13, 17) of the top layer (2) is formed by an electrically conductive foam.

14. Electrode according to claim 10 to 13, **characterized in that** the electrically conductive layer (13, 17) is formed by a printed-on electrically conductive layer.

15. Electrode according to one of claims 10 to 14, **characterized in that** the top layer (2) is constructed as a preferably two-layered film having a first electrically insulating carrier layer (7) and a second electrically conductive layer (8).

16. Electrode according to one of claims 10 to 14, **characterized in that** the electrically conductive side (13, 17) is constructed as a preferably two-layered film having a first electrically insulating carrier layer (7) and a second electrically conductive layer (8).

17. Electrode according to one of claims 18 to 16, **characterized in that** the electrically conductive side (13, 17) of the top layer (2) is galvanically separated from the signal conductor (3) and electrically conductive separate regions of the top layer which may be present are connected electrically.

18. Electrode according to one of claims 10 to 17, **characterized in that** the electrically conductive side (13) of the top layer (2), the signal conductor (3) and the electrically conductive side of the covering element (6, 17) are arranged with vertical spacing from one another, preferably overlapping at least in regions.

19. Electrode according to one of claims 1 to 18, **characterized in that** a skin-friendly, electrically insulating plaster element (14) is arranged on the side of the adhesive layer (4) facing the signal-emitting subject.

20. Electrode according to claim 19, **characterized in that** the plaster element (14) at least partly masks the tapping pad (5), the part of the plaster element (14) masking the tapping pad (5) being electrically conductive in construction on its side facing the tapping pad (5)

21. Electrode according to claim 19 or 20, **characterized in that** the covering element (6) or the electrically insulating carrier layer (7) of the covering element (6) is formed by the plaster element (14).

22. Electrode according to one of claims 1 to 21, **characterized in that** the electrically conductive layer (13, 17, 8) which shields the signal conductor (3) against interference signals is in the form of a Faraday shield or cup.

23. Electrode according to one of claims 1 to 22, **characterized by** a connection element (16) arranged on the electrically conductive layer or connected to this for detachable fixing of the conductor (19) to the electrically conductive layer (13, 17, 8).

24. Electrode according to one of claims 1 to 23, **characterized in that** the signals of the signal conductor (3) and the interference signals of the electrically conductive layer (13, 17, 8) are transmitted via a lead (18) which has two galvanically separated conductors (19, 20).

25. Electrode according to claim 24, **characterized in that** the two conductors (19, 20) of the lead (18) are arranged or constructed coaxially, it being possible for the inner conductor (20) to be brought into contact with the signal conductor (3).

26. Electrode according to one of claims 1 to 25, **characterized in that** the lead (18) has a connection element (21) by means of which the electrically conductive layer (13, 17, 8) and the signal conductor (3) can be brought into contact simultaneously with their corresponding conductors (19, 20) of the lead (18).

## Revendications

1. Électrode médicale (1) pour la dérivation de signaux, en particulier la dérivation de signaux de la peau humaine qui est construire avec au moins deux couches, un conducteur de signaux (3), de préférence métallique, pouvant être relié au sujet émettant des signaux, de préférence sous la forme d'une couche conductrice de signaux, étant disposé entre une couche de recouvrement (2) supérieure et une couche adhésive (4) pouvant venir en contact avec le sujet émettant les signaux, et qui comprend un flacon de prélèvement (5), un élément de recouvrement (6) électriquement isolant étant disposé entre le flacon de prélèvement (5) et le sujet émettant les signaux, **caractérisée en ce qu'**au moins un côté de l'élément de recouvrement (6) de préférence plat est conçu comme étant électriquement conducteur et est séparé du conducteur de signaux (3) de façon galvanique, la au moins une couche électriquement conductrice (13, 17, 8) étant conçue de façon à pouvoir venir en contact avec un conducteur (19) pour dériver des signaux parasites ou bien être reliée à la terre.

2. Electrode selon la revendication 1, **caractérisée en ce que** le côté électriquement conducteur de l'élément de recouvrement (6) est formé d'un élément (8) en forme de grille électriquement conducteur.

3. Electrode selon la revendication 1, **caractérisée en ce que** le côté électriquement conducteur de l'élément de recouvrement (6) est formé d'une mousse (8) électriquement conductrice.

4. Electrode selon l'une des revendications 1 à 3, **caractérisée en ce que** l'élément de recouvrement (6) est formé comme une feuille de préférence à deux couches avec une première couche de support (7) électriquement isolante et une seconde couche (8) électriquement conductrice.

5. Electrode selon l'une des revendications 1 à 4, **caractérisée en ce que** le conducteur de signaux (3) et le côté électriquement conducteur de l'élément de recouvrement (6) sont disposés de façon distante l'un de l'autre verticalement et se chevauchant de préférence au moins sur un secteur.

6. Électrode selon l'une des revendications 1 à 5, **caractérisée en ce que** le conducteur de signaux (3) est relié au sujet émettant les signaux via un gel (11) électriquement conducteur, pouvant être disposé de préférence dans un évidement (10) de la couche adhésive (4).

7. Electrode selon l'une des revendications 1 à 6, **caractérisée en ce que** le flacon de prélèvement (5) comprend un élément de prélèvement (12), de préférence un bouton sphérique riveté, qui se trouve en contact électrique avec le conducteur de signaux (3) et qui est accessible à partir du côté de l'électrode (1) opposé au sujet émettant les signaux.

8. Electrode selon l'une des revendications 1 à 7, **caractérisée en ce que** l'élément de recouvrement (6) est relié, de préférence collé, au flacon de prélèvement (5).

9. Electrode selon l'une des revendications 1 à 8, **caractérisée en ce que** l'élément de recouvrement (6) ou bien la couche de support (7) de l'élément de recouvrement (6) est formé d'un seul tenant avec la couche de recouvrement (2) supérieure de l'électrode (1).

10. Electrode selon l'une des revendications 1 à 9, **caractérisée en ce qu'**au moins le côté (13) de la couche de recouvrement (2) opposé au sujet émettant les signaux est formé de façon électriquement conductrice au moins sur un secteur.

11. Electrode selon la revendication 10, **caractérisée en ce qu'**également le dessous (17) de la couche de recouvrement (2) est formé de façon électriquement conductrice au moins sur un secteur.

12. Electrode selon la revendication 10 ou 11, **caractérisée en ce que** le côté électriquement conducteur (13, 17) de la couche de recouvrement (2) est formé d'un élément électriquement conducteur en forme de grille.

13. Electrode selon la revendication 10 ou 11, **caractérisée en ce que** le côté électriquement conducteur (13, 17) de la couche de recouvrement (2) est formé d'une mousse électriquement conductrice.

14. Electrode selon la revendication 10 à 13, **caractérisée en ce que** la couche électriquement conductrice (13, 17) est formée d'une couche imprimée électriquement conductrice.

15. Electrode selon l'une des revendications 10 à 14, **caractérisée en ce que** la couche de recouvrement (2) est formée comme une feuille de préférence à deux couches avec une première couche de support (7) électriquement isolante et une seconde couche (8) électriquement conductrice.

16. Electrode selon l'une des revendications 10 à 14, **caractérisée en ce que** le côté électriquement conducteur (13, 17) est formé comme une feuille de préférence à deux couches avec une première couche de support (7) électriquement isolante et une seconde couche (8) électriquement conductrice.

17. Electrode selon l'une des revendications 10 à 16, **caractérisée en ce que** le côté électriquement conducteur (13, 17) de la couche de recouvrement (2) est séparé de manière galvanique du conducteur de signaux (3) et, le cas échéant des secteurs distincts existants électriquement conducteurs de la couche de recouvrement sont reliés de préférence électriquement les uns aux autres.

18. Electrode selon l'une des revendications 10 à 17, **caractérisée en ce que** le côté électriquement conducteur (13) de la couche de recouvrement (2), le conducteur de signaux (3) et le côté électriquement conducteur de l'élément de recouvrement (6, 17) sont disposés de façon distante les uns des autres verticalement, de préférence se chevauchant sur au moins un secteur.

19. Electrode selon l'une des revendications 1 à 18, **caractérisée en ce qu'**un élément de pansement (14) non irritant, électriquement isolant est disposé sur le côté de la couche adhésive (4) opposé au sujet émettant des signaux.

20. Electrode selon la revendication 19, **caractérisée en ce que** l'élément de pansement (14) couvre au moins en partie le flacon de prélèvement (5), moyennant quoi la partie de l'élément de pansement (14) recouvrant le flacon de prélèvement (5) est formée comme étant électriquement conductrice sur son côté opposé au flacon de prélèvement (5).

21. Electrode selon la revendication 19 ou 20, **caractérisée en ce que** l'élément de recouvrement (6) ou bien la couche de support (7) électriquement isolante de l'élément de recouvrement (6) est formé de l'élément de pansement (14).

22. Electrode selon l'une des revendications 1 à 21, **caractérisée en ce que** la couche électriquement conductrice (13, 17, 8) qui protège le conducteur de signaux (3) des signaux parasites, est disposée sous la forme d'un écran de Faraday ou bien d'un gobelet.

23. Electrode selon l'une des revendications 1 à 22, **caractérisée par** un élément de raccordement (16) disposé sur la couche électriquement conductrice ou bien relié à celle-ci pour une fixation amovible du conducteur (19) sur la couche électriquement conductrice (13, 17, 8).

24. Electrode selon l'une des revendications 1 à 23, **caractérisée en ce que** la transmission des signaux du conducteur de signaux (3) et des signaux parasites de la couche électriquement conductrice (13, 17, 8) s'effectue via un câble (18), qui comprend deux conducteurs (19, 20) séparés de façon galvanique.

25. Electrode selon la revendication 24, **caractérisée en ce que** les deux conducteurs (19, 20) du câble (18) sont formés ou bien disposés de manière coaxiale, le conducteur interne (20) pouvant être amené en contact avec le conducteur de signaux (3).

26. Electrode selon l'une des revendications 1 à 25, **caractérisée en ce que** le câble (18) comprend un élément de raccordement (21), au moyen duquel la couche électriquement conductrice (13, 17, 8) et le conducteur de signaux (3) peuvent être amenés en contact simultanément avec leur conducteurs correspondants (19, 20) du câble (18).
